(19) Europäisches Patentamt
European Patent Office
Office européen des brevets

(11) Veröffentlichungsnummer: **0 229 400**
**A2**

(12) # EUROPÄISCHE PATENTANMELDUNG

(21) Anmeldenummer: **86118187.3**

(22) Anmeldetag: **31.12.86**

(51) Int. Cl.³: **C 08 G 63/20**
C 08 G 63/48, C 08 G 63/66
C 10 M 119/16, A 61 K 7/00

(30) Priorität: **08.01.86 DE 3600263**

(43) Veröffentlichungstag der Anmeldung:
**22.07.87 Patentblatt 87/30**

(84) Benannte Vertragsstaaten:
**AT CH DE FR GB IT LI**

(71) Anmelder: **HOECHST AKTIENGESELLSCHAFT**
**Postfach 80 03 20**
**D-6230 Frankfurt am Main 80(DE)**

(72) Erfinder: **Stühler, Herbert, Dr.**
**Hochfellnstrasse 12**
**D-8269 Burgkirchen/Alz(DE)**

(72) Erfinder: **Reng, Alwin**
**Im Schulzehnten 22**
**D-6233 Kelkheim (Taunus)(DE)**

(72) Erfinder: **Skrypzak, Werner**
**Eichkopfallee 33**
**D-6237 Liederbach(DE)**

(72) Erfinder: **Quack, Jochen Meinhard, Dr.**
**Wilhelm-Reuter-Strasse 16**
**D-6239 Eppstein/Taunus(DE)**

(54) **Mit Fettsäuren modifizierte Polyester, Verfahren zu deren Herstellung und deren Verwendung zur Erhöhung der Viskosität in tensidhaltigen Präparaten.**

(57) Mit Fettsäuren modifizierte Polyester aus Polyalkylenoxid und dimerisierten Fettsäuren der Formel

$$R\overset{O}{\overset{\|}{C}}O-[(AO)_x-\overset{O}{\overset{\|}{C}}-R^1-\overset{O}{\overset{\|}{C}}-(OA)_x-]_nO\overset{O}{\overset{\|}{C}}R$$

wobei R $C_8$-$C_{22}$-Alkyl oder $C_8$-$C_{22}$-Alkenyl, vorzugsweise $C_{12}$-$C_{18}$-Alkyl, $R^1$ das Alkylgerüst einer dimerisierten Fettsäure mit 22 bis 42, vorzugsweise 34 C-Atomen, A $-C_2H_4-$ oder $-C_3H_6-$, n eine Zahl von 1 bis 5, vorzugsweise 1 bis 3 und x eine Zahl von 20 bis 150, vorzugsweise 23 bis 140 bedeuten. Die Herstellung erfolgt durch gleichzeitige Umsetzung der zugrundeliegenden dimerisierten Fettsäure, Polyalkylenoxid und Fettsäure. Die erhaltenen Polyester werden verwendet als Mittel zur Erhöhung der Viskosität in tensidhaltigen kosmetischen, pharmazeutischen und technischen Präparaten.

**EP 0 229 400 A2**

Croydon Printing Company Ltd.

# Mit Fettsäuren modifizierte Polyester, Verfahren zu deren Herstellung und deren Verwendung zur Erhöhung der Viskosität in tensidhaltigen Präparaten

Wasserhaltige Lösungen, Emulsionen oder Suspensionen von Tensiden werden häufig aus anwendungstechnischen Gründen auf höhere Viskositätswerte eingestellt. Solche Mischungen werden beispielsweise zur Formulierung von kosmetischen, pharmazeutischen und technischen Präparaten verwendet. Die hohe Viskosität solcher Mischungen erleichtert die Handhabung, beispielsweise bei viskosen Haarshampooeinstellungen, die dann nicht so leicht von den Händen und den Haaren fließen. Außerdem ermöglichen sie eine Reduzierung der Gebrauchsmenge, beispielsweise bei Geschirrspülmitteln oder eine einfachere Dosierung, beispielsweise bei der Anwendung von Wäscheweichspülmitteln. Zusätzlich kann bei der Produktion die Abfüllung erleichtert werden. Weitere Vorteile ergeben sich beim Einsatz von viskositätserhöhenden Mitteln vor allem in der Verbesserung der Lagerstabilität von wasserhaltigen Tensidsystemen mit wasserunlöslichen Wirkstoffen, z.B. bei Antischuppenshampoos mit Zinkpyrithione als Wirkstoff.

Um die rheologischen Eigenschaften von wasserhaltigen Tensidsystemen zu beeinflussen, werden in der Fachliteratur eine Vielzahl sogenannter Verdickungsmittel vorgeschlagen.

Bekannt sind Elektrolyte, wie beispielsweise Kochsalz zur Verdickung von Alkylethersulfaten bei der Herstellung von Haar- und Hautreinigungsmitteln. Auch Zelluloseether, Fettpolyethylenglykolmono- oder -diester, Natriumalginat, Fettsäurealkanolamide, hochdisperse amorphe Kieselsäure, Polymere und ähnliche Substanzen können allein oder in Kombination miteinander eingesetzt werden.

Diese sogenannten Verdickungsmittel zeigen jedoch bei der Anwendung in wasserhaltigen Tensidsystemen eine Reihe von Nachteilen. So sind beispielsweise mit Zelluloseethern hergestellte Formulierungen bakterienanfällig und ergeben bei der Anwendung unangenehme "fadenziehende" Gele. Anorganische Verdickungsmittel hingegen, wie beispielsweise hochdisperse amorphe Kieselsäure, führen zu Niederschlägen in den Fertigformulierungen. Besonders erwähnenswert ist der Einsatz von Elektrolyten wie Kochsalz zur Viskositätserhöhung von Alkylethersulfatlösungen. Diese können aber bei der Lagerung der Fertigpräparate in Metallbehältern zu unangenehmen Korrosionserscheinungen führen.

Es wurde nun gefunden, daß Polyester, hergestellt aus Polyethylenglykolen und dimeren Fettsäuren, deren OH-Endgruppen mit Monocarbonsäuren verschlossen sind, besonders gut zur Viskositätserhöhung von wasserhaltigen Tensidsystemen geeignet sind und die oben geschilderten Nachteile nicht aufweisen.

Gegenstand der Erfindung sind mit Fettsäuren modifizierte Polyester aus Polyalkylenoxid und dimerisierten Fettsäuren der Formel

$$R\overset{O}{\overset{\|}{C}}O-[(AO)_x-\overset{O}{\overset{\|}{C}}-R^1-\overset{O}{\overset{\|}{C}}-(OA)_x-]_n O\overset{O}{\overset{\|}{C}}R$$

wobei R $C_8$-$C_{22}$-Alkyl oder $C_8$-$C_{22}$-Alkenyl, vorzugsweise $C_{12}$-$C_{18}$-Alkyl, $R^1$ das Alkylgerüst einer dimerisierten Fettsäure mit 22 bis 42, vorzugsweise 34 C-Atomen, A $-C_2H_4-$ oder $-C_3H_6-$, n eine Zahl von 1 bis 5, vorzugsweise 1 bis 3 und x eine Zahl von 20 bis 150, vorzugsweise 23 bis 140 bedeuten.

Diese Verbindungen werden hergestellt durch gemeinsame Kondensation einer dimerisierten Fettsäure der Formel

$$HOOC-R^1-COOH$$

mit einem Polyalkylenoxid der Formel

$$H(OA)_xOH$$

und einer Fettsäure der Formel

$$R-COOH$$

wobei $R^1$, R, A und x die obengenannten Bedeutungen haben.

Als dimerisierte Fettsäuren kommen vorzugsweise die Produkte in Frage, die unter der Bezeichnung Pripol (Unichema) im Handel erhältlich sind. Diese dimerisierten Fettsäuren enthalten im wesentlichen lineare und zyklische Verbindungen, vermutlich der folgenden Struktur:

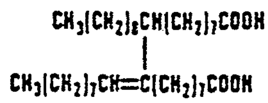

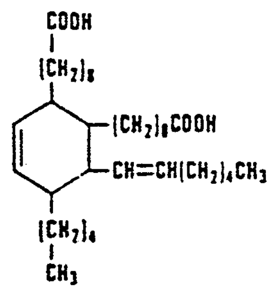

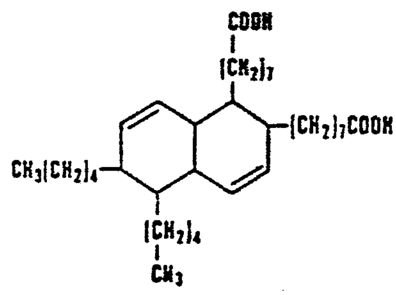

Daneben enthalten diese Produkte auch noch Anteile von trimeren und höher kondensierten Fettsäuren. Als Polyalkylenoxide kommen vorzugsweise Polyethylenoxide in Betracht, beispielsweise Polyethylenoxide mit einer Molmasse von 800, 1000, 3000 oder 6000. Als Fettsäuren kommen $C_8$-$C_{22}$-Fettsäuren in Frage, bevorzugt $C_{12}$-$C_{18}$-gesättigte Fettsäuren und isomere Fettsäuren wie Isostearinsäure.

Diese Verbindungen werden gemeinsam unter saurer Katalyse miteinander verestert. Die Molverhältnisse der drei Ausgangsverbindungen betragen, abhängig vom gewünschten Viskositätsgrad bei der Verwendung dieser Polyester, 1 : 2 : 2 oder 2 : 3 : 2 oder 4 : 5 : 2 (dimerisierte Fettsäure : Polyalkylenoxid : Fettsäure). Die Veresterung wird bei einer Temperatur von 150 bis 200°C, vorzugsweise 180 bis 190°C und bei einem Druck von 80 bis 200 mbar durchgeführt bei gleichzeitiger Entfernung des gebildeten Reaktionswassers. Während der Reaktion wird das Reaktionsgemisch mit Stickstoff überlagert. Als saure Katalysatoren kommen die bekannten Katalysatoren und Katalysatorsysteme in Frage wie beispielsweise Schwefelsäure, Lewis-Säuren oder die Mischungen Dodecylbenzolsulfonsäure/Unterphosphorige Säure oder Methansulfonsäure/Unterphosphorige

Säure. Die Reaktion wird durch laufende Bestimmung der Säurezahl und durch Messung der Menge an abdestilliertem Wasser verfolgt. Das Umsetzungsprodukt soll eine Säurezahl von höchstens 3, vorzugsweise höchstens 1 aufweisen. Die Reaktionszeit zum Erreichen dieser Säurezahl hängt in erster Linie von der Temperatur ab. Sie beträgt im allgemeinen 18 bis 30 Stunden. Nach Erreichen der angestrebten Säurezahl wird zweckmäßigerweise möglichst rasch auf Raumtemperatur abgekühlt. Man erhält die Polyester als leicht gelb gefärbte Produkte.

Mit diesen Polyestern lassen sich klare, hochviskose Einstellungen der gängigen nichtionischen und/oder anionischen und/oder amphoteren Tensiden in einfacher Weise herstellen, z.B. kosmetische, pharmazeutische und technische Präparate. Hierbei können die erfindungsgemäßen Polyester mit den üblichen Komponenten dieser Präparate kombiniert werden. So können sie beispielsweise in Haarpflegemittel, Hautreinigungsmittel, wie beispielsweise Schaum- und Duschbadpräparate, Zahnreinigungsmittel, Haarverformungsmittel, Salbe, Geschirrspülmittel, Kraftfahrzeugreinigungsmittel, Haushaltsreinigungsmittel und andere technische Reinigungspräparate eingearbeitet werden. Der mengenmäßige Anteil der beschriebenen Polyester in den verschiedensten tensidhaltigen Präparaten kann je nach der gewünschten Viskosität zwischen 0,1 bis etwa 10 %, vorzugsweise jedoch zwischen 1 und 5 %, bezogen auf das Gewicht der Fertigformulierung, schwanken. Die Einarbeitung dieser Polyester in die zu verdickenden Lösungen, Suspensionen oder Emulsionen erfolgt in an sich bekannter Weise durch Auflösen des Polyesters, evtl. unter Erwärmen, in dem Wasser bzw. in der wasserhaltigen Phase.

Fertigpräparate, die unter Verwendung der erfindungsgemäßen Polyester auf eine höhere Viskosität eingestellt wurden, zeigen folgende Vorteile: Es wird eine Viskositätserhöhung von gelösten, suspendierten oder emulgierten wasserhaltigen

Lösungen von anionischen und/oder nichtionischen und/oder amphoteren Tensiden erreicht. Die Polyester zeigen beim Einsatz als Verdickungsmittel, beispielsweise bei Alkylethersulfatlösungen, im Vergleich zu dem häufig eingesetzten Verdickungsmittel Kochsalz eine verringerte Korrosionswirkung. Bei Verwendung dieser Polyester zur Herstellung von hochviskosen Haarshampoos wird nach der Anwendung auf dem Haar ein zusätzlicher Konditioniereffekt erzielt, d.h. das Haar ist besser kämmbar und die Formgebung der Frisur wird erleichtert. Die mit den erfindungsgemäßen Polyestern hergestellten wäßrigen Tensidlösungen sind im Vergleich zu Formulierungen, die mit Zelluloseethern bzw. Verdickungsmitteln nativer Provenienz, wie Agar-Agar, Gelatine verdickt sind, wesentlich weniger gegen Mikroorganismen, wie beispielsweise Bakterien, anfällig. Es können klare, tensidhaltige, hochviskose Fertigpräparate hergestellt werden, während anorganische Verdickungsmittel, wie beispielsweise hochdisperse amorphe Kieselsäure oder Natriumaluminiumsilikat zu Trübungen und Ausfällungen in den genannten Systemen führen. Die beschriebenen Polyester erhöhen die Lagerstabilität dieser Präparate und die Verteilbarkeit bzw. Handhabung dieser Präparate bei der Anwendung wird erleichtert. Zusätzlich wird ein angenehmeres Hautgefühl nach der Anwendung erreicht. Durch den Einbau der Polyester in wasserhaltige Tensidformulierungen wird außerdem das Schaumbild positiv beeinflußt, d.h. es entsteht ein cremiger, sahniger Schaum mit einem günstigeren anwendungstechnischen Verhalten als bei der Verwendung der üblichen Verdickungsmittel.

Beispiel 1

150 g Polyethylenglykol 3000, 23,0 g Dimersäure PRIPOL 1015, 6,1 g Isostearinsäure und 0,45 g Methansulfonsäure und 0,45 g Unterphosphorige Säure (50 %ig) als Katalysatoren werden in einem 1-Liter-Vierhalskolben vorgelegt.

Das Gemisch wird unter Rühren und Stickstoffüberdeckung auf 180°C aufgeheizt und Vakuum angelegt. Der Druck wird durch Überleiten von Stickstoff auf 100 mbar eingestellt. Nach 18 Stunden Reaktionszeit wird eine Probe zur Bestimmung der Restsäurezahl entnommen. Ist die Säurezahl kleiner als 1, wird das Produkt auf 80°C gekühlt und abgefüllt.

Beispiel 2

300 g Polyethylenglykol 6000, 23,0 g Dimersäure PRIPOL 1015, 6,1 g Isostearinsäure und 0,82 g Methansulfonsäure und 0,82 g Unterphosphorige Säure (50 %ig) als Katalysatoren werden in einem 1-Liter-Vierhalskolben vorgelegt.

Das Gemisch wird unter Rühren und Stickstoffüberdeckung auf 180°C aufgeheizt und Vakuum angelegt. Der Druck wird durch Überleiten von Stickstoff auf 100 mbar eingestellt. Nach 21 Stunden Reaktionszeit wird eine Probe zur Bestimmung der Restsäurezahl entnommen. Die Säurezahl war kleiner als 1; das Produkt wurde auf 80°C gekühlt und abgefüllt.

Beispiel 3

300 g Polyethylenglykol 6000, 23,0 g Dimersäure PRIPOL 1015, 15,2 g Isostearinsäure und 0,82 g Methansulfonsäure und 0,82 g Unterphosphorige Säure (50 %ig) als Katalysatoren werden in einem 1-Liter-Vierhalskolben vorgelegt.

Das Gemisch wird unter Rühren und Stickstoffüberdeckung auf 170°C aufgeheizt und Vakuum angelegt. Der Druck wird durch Überleiten von Stickstoff auf 100 mbar eingestellt. Nach 25 Stunden Reaktionszeit wird eine Probe zur Bestimmung der Restsäurezahl entnommen. Die Säurezahl war kleiner als 1; das Produkt wurde auf 80°C gekühlt und abgefüllt.

Beispiel 4

3,7 g des Polyesters gemäß Beispiel 1 werden in 47 g destilliertem Wasser unter Rühren und Erwärmen gelöst. Anschließend wird in diese Lösung 50 g einer 28 %igen Lösung eines Natriumalkylethersulfats der Formel

$$RO-(CH_2CH_2O)_2-SO_3^{\oplus}Na^{\oplus} \quad z= 1 - 5; \; R= C_8-C_{18}\text{-Alkyl}$$

gegeben, in die vorher 0,2 g Parfümöl gegeben wurden.

Das so hergestellte Shampoo zeigt eine Viskosität von 2400 mPas, gemessen im Brookfield-Reaktionsviskosimeter RVT bei 20°C.

Beispiel 5 - Duschbad

50 g einer 40 %igen wäßrigen Acylamidoalkylethersulfattriethanolaminsalzlösung werden mit 0,3 g Parfümöl und 56,5 g destilliertem Wasser verdünnt. Anschließend werden in diese Mischung 3,2 g des in Beispiel 1 genannten Polyesters unter langsamem Rühren und gleichzeitigem Erwärmen aufgelöst.

Das Duschbadpräparat besitzt bei 20°C eine Viskosität von 1900 mPas.

Beispiel 6 - Geschirrspülmittel

10 g sekundäres Alkansulfonat-Natriumsalz werden zusammen mit 3 g Alkylethersulfat-Natriumsalz in 95 g destilliertem Wasser gelöst und dieser Mischung werden anschließend 2 g des in Beispiel 1 genannten Polyesters unter Rühren und Erwärmen zugesetzt.

Das Geschirrspülmittel besitzt eine Viskosität von 850 mPas.

Beispiel 7 - Schaumbad

30 g einer 28 %igen Alkyldiglykolethersulfatlösung werden mit 20 g eines 30 %igen Sulfobernsteinsäureester-Natriumsalzes gemischt und in diese Lösung werden anschließend 44,5 g Wasser und 0,5 g Parfümöl eingerührt. Anschließend erfolgt der Zusatz von 5,0 g des in Beispiel 1 beschriebenen Polyesters.

Die Formulierung besitzt bei 20°C die Viskosität von 2320 mPas.

Beispiel 8 - Gesichtswaschlotion

30 g einer 40 %igen wäßrigen Acylamidoalkylethersulfattriethanolaminsalzlösung werden mit 0,3 g Parfümöl und 13 g einer 30 %igen Lauroylsarcosidnatriumsalzlösung gemischt. Zusätzlich werden noch 0,2 g Allantoin und 51,5 g Wasser in die vorstehende Mischung eingerührt. Anschließend werden dieser Lösung 5,0 g des in Beispiel 1 beschriebenen Polyesters zugesetzt.

Die Gesichtswaschlotion besitzt bei +20°C eine Viskosität von 1229 mPas.

Ansprüche: HOE 86/F 001

1. Mit Fettsäuren modifizierte Polyester aus Polyalkylenoxid und dimerisierten Fettsäuren der Formel

$$R\overset{O}{\overset{\|}{C}}O-[(AO)_x-\overset{O}{\overset{\|}{C}}-R^1-\overset{O}{\overset{\|}{C}}-(OA)_x-]_nO\overset{O}{\overset{\|}{C}}R$$

wobei R $C_8$-$C_{22}$-Alkyl oder $C_8$-$C_{22}$-Alkenyl, vorzugsweise $C_{12}$-$C_{18}$-Alkyl, $R^1$ das Alkylgerüst einer dimerisierten Fettsäure mit 22 bis 42, vorzugsweise 34 C-Atomen, A $-C_2H_4-$ oder $-C_3H_6-$, n eine Zahl von 1 bis 5, vorzugsweise 1 bis 3 und x eine Zahl von 20 bis 150, vorzugsweise 23 bis 140 bedeuten.

2. Verfahren zur Herstellung der Verbindungen nach Anspruch 1, dadurch gekennzeichnet, daß man eine dimerisierte Fettsäure der Formel

$$HOOC-R^1-COOH$$

gemeinsam mit einem Polyalkylenoxid der Formel

$$H(OA)_xOH$$

und einer Fettsäure der Formel

$$RCOOH$$

umsetzt.

3. Verwendung der Verbindungen nach Anspruch 1 zur Erhöhung der Viskosität in tensidhaltigen kosmetischen, pharmazeutischen und technischen Präparaten.

Patentansprüche Österreich:

1. Verfahren zur Herstellung von mit Fettsäuren modifizierten Polyestern aus Polyalkylenoxid und dimerisierten Fettsäuren der Formel

$$R\overset{O}{\overset{\|}{C}}O-[(AO)_x-\overset{O}{\overset{\|}{C}}-R^1-\overset{O}{\overset{\|}{C}}-(OA)_x-]_n O\overset{O}{\overset{\|}{C}}R$$

wobei R $C_8$-$C_{22}$-Alkyl oder $C_8$-$C_{22}$-Alkenyl, vorzugsweise $C_{12}$-$C_{18}$-Alkyl, $R^1$ das Alkylgerüst einer dimerisierten Fettsäure mit 22 bis 42, vorzugsweise 34 C-Atomen, A $-C_2H_4-$ oder $-C_3H_6-$, n eine Zahl von 1 bis 5, vorzugsweise 1 bis 3 und x eine Zahl von 20 bis 150, vorzugsweise 23 bis 140 bedeuten, dadurch gekennzeichnet, daß man eine dimerisierte Fettsäure der Formel

$$HOOC-R^1-COOH$$

gemeinsam mit einem Polyalkylenoxid der Formel

$$H(OA)_xOH$$

und einer Fettsäure der Formel

$$RCOOH$$

umsetzt.

2. Verwendung der nach Anspruch 1 hergestellten Verbindungen zur Erhöhung der Viskosität in tensidhaltigen kosmetischen, pharmazeutischen und technischen Präparaten.